Europäisches Patentamt

**European Patent Office**

**Office européen des brevets**

(19)

(11) Publication number: **0 179 518**
**A1**

(12)

# EUROPEAN PATENT APPLICATION

(21) Application number: **85201615.3**

(22) Date of filing: **07.10.85**

(51) Int. Cl.⁴: **A 61 F 5/47**

(30) Priority: **16.10.84 NL 8403150**

(43) Date of publication of application:
**30.04.86 Bulletin 86/18**

(84) Designated Contracting States:
**AT BE CH DE FR GB IT LI NL SE**

(71) Applicant: **AKZO N.V.**
**Velperweg 76**
**NL-6824 BM Arnhem(NL)**

(72) Inventor: **De Leede, Leonardus G.J. Dr.**
**Aalbersestraat 36**
**NL-5344 SV Oss(NL)**

(74) Representative: **Hermans, Franciscus G.M. et al,**
**Postbus 20**
**NL-5340 BH Oss(NL)**

(54) **IUD extractor thread.**

(57) The present invention relates to an intrauterine device
(IUD) which is provided with a so-called extractor thread.

Croydon Printing Company Ltd.

1

# IUD EXTRACTOR THREAD

The present invention relates to an intrauterine device (IUD) which is provided with a so-called extractor thread.

It is known and customary for IUD's fitted in the uterus for the purpose of preventing pregnancy to be provided with a so-called extractor thread. This extractor thread, which, when the IUD is correctly fitted, has a length such that it is situated partly in the "sterile" uterus and partly in the "non-sterile" vagina, and ensures that the IUD can, when desired, be rapidly extracted and also makes it substantially simpler to check that the IUD is still in position in the uterus.

However, the presence of a thread of this kind also has disadvantages.

Bacteriological tests have shown that the occurrence of infections near, at or in the uterus is significantly higher for the wearer of an IUD than in users of other forms of contraception. In addition, it was found that P.I.D. (pelvic inflammatory disease) occured significantly less frequently when use was made of an IUD without an extractor thread than in the case of the use of an IUD with an extractor thread. The conclusion thus appears to be that the extractor thread is in many cases responsible for carrying microorganisms to vulnerable or injured parts.

In order to prevent the possibililty of direct infection, it occurs in practice that the doctor fitting the IUD will dip the entire IUD into a solution of an antiseptic. The extractor thread of the IUD is then also to a greater of lesser extent impregnated with the antiseptic. The effect of this treatment is however of short duration and is generally too short to justify speaking of an effective long term treatment.

The introduction of an antiseptic, particulary into the vagina and cervix, in this way also increases the likelihood of undesirable side effects through action on the bacteria flora, while the possible systemic effects of an antiseptic applied locally in this manner are also not yet fully known.

It has now been found that the problem of the occurrence of infections could be solved in many cases by providing the IUD with an extractor thread which is coated with a thin layer of metallic silver.

This extractor thread coated with metallic silver will, during the entire time that the IUD is present in the uterus, prevent infections arising through the carrying of microorganisms from the vagina (and cervix), and in this way makes a considerable contribution towards the reduction of the chances of infections.

The extractor thread must usually be very thin. It preferably has a diameter not larger than about 0.2 to 0.3 mm. In addition, the thread must have a high degree of flexibility and should obviously have a certain strength, which should not or should practically not be affected by a lenghty stay in the cervix and uterus.

A thin layer of metallic silver does not influence the flexiblility of the thread, or does so scarcely at all, while the strength of the thread is certainly as great as that of uncoated thread.

The core of the thread may be of any suitable material. However, a polymer material meeting the requirements in respect of strength and flexibility is preferably used. Examples of suitable threads which can be provided with a layer of metallic silver include, among others, nylon 6, nylon 66, polyethylene and polypropylene.

A nylon thread coated with metallic silver, which can be effectively used for the present invention, is supplied by Swift Textile Metalizing Corp., Hartford, Conn., USA. The thread has a total weight of about 40 mg per meter, of which from 3 to 20% consists of metallic silver. The coated thread has an average diameter of about 0.2 mm.

The IUD provided with an extractor thread according to this invention may be further improved, if desired, by dipping the entire IUD including the thread into a solution of an antiseptic material just before the insertion of the IUD into the uterus.

More preferably the IUD is provided with a thin layer or coating consisting essentially of an antiseptic substance contained in a polymeric material. The antiseptic substance will then be released from the polymer either by diffusion or by dissolution of the polymer (including the antiseptic) in the surrounding body-liquid.

Said thin layer or coating can be obtained by dipping the IUD in a solution of the polymer material and the antiseptic substance in a suitable liquid such as water or alcohol, followed by removing the solvent from the coating. Preferred antiseptic substances in this respect are benzalkonium chloride, sorbic acid, chlorhexidine and salts thereof, and iodine. Preferred polymers are water-soluble polymers such as hydroxypropylmethylcellulose (HPMC) and especially polyvinyl-pyrrolidone (PVP).

1

## CLAIMS

1.     Intrauterine device (IUD) provided with a so-called extractor thread, characterised in that this extractor thread is coated with metallic silver.

2.     The IUD of claim 1, provided with a thin layer or coating consisting essentially of an antiseptic substance, contained in a polymer material.

European Patent
Office

**EUROPEAN SEARCH REPORT**

**0179518**

Application number

EP 85 20 1615

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-1 896 071  (CLARK)<br>* Page 1, lines 74-82,85-92 figure 11 * | 1 | A 61 F    5/47 |
| X | US-A-2 122 579  (MECKSTROTH)<br>* Page 2, left-hand column, lines 62-69; figure 23 * | 1 | |
| A | | 2 | |
| A | US-A-3 467 089  (HASSON)<br>* Column 2, lines 52-59; figures * | 1,2 | |
| A | DE-A-2 820 525  (BAYER)<br>* Page 3, lines 1-7 * | 1 | |
| A | US-A-4 034 749  (VON KESSERÜ et al.)<br>* Column 7, lines 29-34,56-60 * | 1,2 | TECHNICAL FIELDS SEARCHED (Int. Cl.4)<br><br>A 61 F |
| A | US-A-4 233 968  (SHAW, Jr.)<br>* Column  9, lines 43-48; column 10, lines 30-38 * | 1,2 | |
| A | US-A-3 760 806  (LEEPER)<br>* Column 9, lines 12-20 * | 2 | |

The present search report has been drawn up for all claims

| Place of search<br>THE HAGUE | Date of completion of the search<br>17-01-1986 | Examiner<br>GLAS J. |
|---|---|---|